(19) **Europäisches Patentamt** / European Patent Office / Office européen des brevets

(11) **EP 2 486 084 B1**

(12) **EUROPÄISCHE PATENTSCHRIFT**

(45) Veröffentlichungstag und Bekanntmachung des Hinweises auf die Patenterteilung:
**23.03.2016 Patentblatt 2016/12**

(21) Anmeldenummer: **10760367.2**

(22) Anmeldetag: **05.10.2010**

(51) Int Cl.:
*C08J 3/24* (2006.01)          *C08J 3/12* (2006.01)
*C08F 6/26* (2006.01)          *A61L 15/60* (2006.01)
*A61L 15/22* (2006.01)

(86) Internationale Anmeldenummer:
**PCT/EP2010/064817**

(87) Internationale Veröffentlichungsnummer:
**WO 2011/042429 (14.04.2011 Gazette 2011/15)**

(54) **VERFAHREN ZUR NACHBEFEUCHTUNG OBERFLÄCHENNACHVERNETZTER WASSERABSORBIERENDER POLYMERPARTIKEL**

PROCESS FOR REMOISTURIZING SURFACE-POSTCROSSLINKED WATER-ABSORBING POLYMER PARTICLES

PROCÉDÉ DE POST-HUMIDIFICATION DE PARTICULES POLYMÈRES POST-RÉTICULÉES EN SURFACE, QUI ABSORBENT L'EAU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.10.2009 US 250036 P**

(43) Veröffentlichungstag der Anmeldung:
**15.08.2012 Patentblatt 2012/33**

(73) Patentinhaber: **BASF SE**
**67056 Ludwigshafen (DE)**

(72) Erfinder:
• **FUNK, Rüdiger**
  **65527 Niedernhausen (DE)**
• **SCHRÖDER, Jürgen**
  **67071 Ludwigshafen (DE)**
• **PFEIFFER, Thomas**
  **67459 Böhl-Iggelheim (DE)**
• **WEISMANTEL, Matthias**
  **63637 Jossgrund (DE)**

(56) Entgegenhaltungen:
EP-A1- 2 135 669          US-A1- 2005 013 992
US-A1- 2005 222 344

EP 2 486 084 B1

**EP 2 486 084 B1**

**Beschreibung**

Die vorliegende Erfindung betrifft ein Verfahren zur Herstellung wasserabsorbierender Polymerpartikel, wobei oberflächennachvernetzte wasserabsorbierende Polymerpartikel nachbefeuchtet und pneumatisch gefördert werden und wobei der zeitliche Abstand zwischen Nachbefeuchtung und pneumatischer Förderung weniger als eine Stunde beträgt.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm$^2$ (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49.2 g/cm$^2$ (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 g/cm$^2$ (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der wasserabsorbierenden Polymerpartikel kovalente Bindungen bilden können.

Die wasserabsorbierenden Polymerpartikel weisen nach der thermischen Oberflächennachvernetzung oft einen Feuchtegehalt von weniger als 1 Gew.-% auf. Dadurch erhöht sich die Neigung der Polymerpartikel zur statischen Aufladung. Die statische Aufladung der Polymerpartikel beeinflusst die Dosiergenauigkeit beispielsweise bei der Windelherstellung. Dieses Problem wird üblicherweise durch Einstellung eines definierten Feuchtegehalts durch Zusatz von Wasser oder wässrigen Lösungen gelöst (Nachbefeuchtung),

Verfahren zur Nachbefeuchtung werden beispielsweise in WO 98/49221 A1 und EP 0 780 424 A1 offenbart.

Wasserabsorbierende Polymerpartikel können mittels pneumatischer Fördersysteme transportiert werden,

Verfahren zur pneumatischen Förderung werden beispielsweise in WO 2007/104657 A2, WO 2007/104673 A2 und WO 2007/104676 A1 beschrieben.

US 2005/ 0013 992 beschreibt die Beschichtung wasserabsorbierender Polymerpartikel mit einer Schale aus einem vernetzten, kationischen Polymer.

US 2005/0222344 beschreibt die Nachbefeuchtung mittels feuchter Luft, beispielsweise während der pneumatischen Förderung.

EP 2 135 669 A1 beschreibt die Beschichtung wasserabsorbierender Polymerpartikel mit einen Schmiermittel und einem Permeabilitätsverbesserer und die anschließende pneumatische Förderung.

Aufgabe der vorliegenden Erfindung war die Bereitstellung eines verbesserten Verfahrens zur Herstellung wasserabsorbierender Polymerpartikel, insbesondere die Vermeidung und/oder eine verbesserte Abtrennung zu kleiner Polymerpartikel.

Gelöst wurde die Aufgabe durch ein Verfahren zur Herstellung wasserabsorbierender Polymerpartikel durch Polymerisation einer Monomerlösung oder -suspension, enthaltend a) mindestens ein ethylenisch ungesättigtes, säuregruppentragendes Monomer, das zumindest teilweise neutralisiert sein kann, b) mindestens einen Vernetzer, c) mindestens einen Initiator, d) optional ein oder mehrere mit den unter a) genannten Monomeren copolymerisierbare ethylenisch ungesättigte Monomere und e) optional ein oder mehrere wasserlösliche Polymere, umfassend Trocknung, Mahlung, Klassierung, und Oberflächennachvernetzung, wobei i) die oberflächennachvernetzten Polymerpartikel nachbefeuchtet, ii) die nachbefeuchteten Polymerpartikel pneumatisch gefördert und iii) optional die nachbefeuchteten Polymerpartikel klassiert werden, dadurch gekennzeichnet, dass zur Nachbefeuchtung i) ein Mischer mit rotierenden Mischwerkzeugen eingesetzt wird und der zeitliche Abstand zwischen der Nachbefeuchtung i) und der pneumatischen Förderung ii) weniger als eine Stunde beträgt.

Der Nachbefeuchtung i) wird üblicherweise durch Zusatz einer wässrigen Flüssigkeit in geeigneten Mischeinrichtungen durchgeführt. Geeignete wässrige Flüssigkeiten sind Wasser, wässrige Lösungen und wässrige Dispersionen. Vorteilhaft werden Mischer mit schnell laufenden Mischwerkzeugen eingesetzt, da hierdurch die Verklumpungsneigung der wasserabsorbierenden Polymerpartikel minimiert wird. Weitere Einflussgrößen der Verklumpungsneigung sind die Temperatur der wasserabsorbierenden Polymerpartikel und die Ionenstärke der zur Nachbefeuchtung eingesetzten wässrigen Lösung. Die Verklumpungsneigung nimmt mit steigender Temperatur und steigender Ionenstärke ab.

Die Temperatur der der Nachbefeuchtung i) zugeführten wasserabsorbierenden Polymerpartikel beträgt daher vorzugsweise von 40 bis 80°C, besonders bevorzugt von 45 bis 75°C, ganz besonders bevorzugt von 50 bis 70°C.

Die Mischer mit rotierenden Mischwerkzeugen werden gemäß der Lage der Rotationsachse zur Produktstromrichtung in Vertikalmischer und Horizontalmischer unterteilt. Vorteilhaft werden Horizontalmischer zur Nachbefeuchtung verwendet.

Geeignete Horizontalmischer mit bewegten Mischwerkzeugen sind beispielsweise Schneckenmischer, Scheibenmischer, Schaufelmischer, Schraubenbandmischer und Durchlaufmischer. Die wässrige Flüssigkeit kann sowohl in Hochgeschwindigkeitsmischern als auch in Mischern mit geringer Rührgeschwindigkeit aufgesprüht werden. Ein bevorzugter Horizontalmischer ist der Durchlaufmischer. Geeignete Durchlaufmischer sind beispielsweise bei Gebrüder Ruberg GmbH & Co KG, Nieheim, DE, erhältlich.

Die Innenwand des Horizontalmischers weist gegenüber Wasser einen Randwinkel von vorzugsweise weniger als 80°, besonders bevorzugt von weniger als 65°, ganz besonders bevorzugt von weniger als 50°, auf. Der Randwinkel ist ein Maß für das Benetzungsverhalten und wird gemäß DIN 53900 gemessen.

Vorteilhaft werden Horizontalmischer eingesetzt, deren produktberührte Innenwand aus einem nichtrostenden Stahl ist. Nichtrostende Stähle weisen üblicherweise einen Chromgehalt von 10,5 bis 13 Gew.-% Chrom auf. Der hohe Chromanteil führt zu einer schützenden Passivierung aus Chromdioxid an der Stahloberfläche. Weitere Legierungsbestandteile erhöhen die Korrosionsbeständigkeit und verbessern die mechanischen Eigenschaften.

Besonders geeignete Stähle sind austenitische Stähle mit beispielsweise mindestens 0,08 Gew.-% Kohlenstoff. Vorteilhaft enthalten die austenitischen Stähle neben Eisen, Kohlenstoff, Chrom, Nickel und optional Molybdän noch weitere Legierungsbestandteile, vorzugsweise Niob oder Titan.

Die bevorzugten nichtrostenden Stähle sind Stähle mit der Werkstoffnummer 1.45xx gemäß der DIN EN 10020, wobei xx eine natürliche Zahl zwischen 0 und 99 sein kann.

Besonders bevorzugte Werkstoffe sind die Stähle mit den Werkstoffnummern 1.4541 und 1.4571, insbesondere Stahl mit der Werkstoffnummer 1.4571.

Vorteilhaft ist die produktberührte Innenwand des Horizontalmischers poliert. Polierte nichtrostende Stahloberflächen weisen eine niedrigere Rauhigkeit und einen niedrigeren Randwinkel gegenüber Wasser auf als matte oder aufgeraute Stahloberflächen.

Die Verweilzeit im Horizontalmischer beträgt vorzugsweise von 1 bis 180 Minuten, besonders bevorzugt von 2 bis 60 Minuten, ganz besonders bevorzugt von 5 bis 20 Minuten.

Die Umfangsgeschwindigkeit der Mischwerkzeuge im Horizontalmischer beträgt vorzugsweise von 0,1 bis 10 m/s, besonders bevorzugt von 0,5 bis 5 m/s, ganz besonders bevorzugt von 0,75 bis 2,5 m/s.

Die oberflächennachvernetzten wasserabsorbierenden Polymerpartikel werden im Horizontalmischer mit einer Geschwindigkeit bewegt, die einer Froude-Zahl von vorzugsweise 0,01 bis 6, besonders bevorzugt 0,05 bis 3, ganz besonders bevorzugt 0,1 bis 0,7, entspricht.

Für Mischer mit horizontal gelagertem Mischwerkzeugen ist die Froude-Zahl wie folgt definiert:

$$Fr = \frac{\omega^2 r}{g}$$

mit

r: Radius des Mischwerkzeugs
ω: Kreisfrequenz
g: Erdbeschleunigung:

Der Füllgrad des Horizontalmischers beträgt vorzugsweise von 30 bis 80%, besonders bevorzugt von 40 bis 75%, ganz besonders bevorzugt von 50 bis 70%.

Die wässrige Flüssigkeit wird vorzugsweise mittels einer Zweistoffdüse, besonders bevorzugt mittels einer innenmischenden Zweistoffdüse, aufgesprüht.

Zweistoffdüsen ermöglichen eine Zerstäubung in feine Tröpfchen bzw. einen Sprühnebel. Als Zerstäubungsform wird ein kreisförmiger oder auch elliptischer Voll- oder Hohlkegel ausgebildet. Zweistoffdüsen können außenmischend oder innenmischend gestaltet werden. Bei den außenmischenden Zweistoffdüsen verlassen Flüssigkeit und Zerstäubergas den Düsenkopf über separate Öffnungen. Sie werden erst nach dem Austritt aus der Sprühdüse im Sprühstrahl gemischt. Dies ermöglicht eine im weiten Bereich unabhängige Regelung von Tropfengrößenverteilung und Durchsatz. Der Sprühkegel der Sprühdüse kann über die Luftkappenstellung eingestellt werden. Bei der innenmischenden Zweistoffdüse werden Flüssigkeit und Zerstäubergas innerhalb der Sprühdüse vermischt und das Zweiphasengemisch verlässt den Düsenkopf über dieselbe Bohrung (bzw. über mehrere parallel geschaltete Bohrungen). Bei der innenmischenden Zweistoffdüse sind die Mengen- und Druckverhältnisse stärker gekoppelt als bei der außenmischenden Sprühdüse. Geringe Veränderungen im Durchsatz führen deshalb zur Änderung der Tropfengrößenverteilung. Die Anpassung an den gewünschten Durchsatz erfolgt über den gewählten Querschnitt der Düsenbohrung.

Als Zerstäubergas kommen Pressluft, Gas oder Dampf von 0,5 bar und mehr in Frage. Die Tröpfchengröße kann individuell über das Verhältnis von Flüssigkeit zu Zerstäubergas sowie Gas- und Flüssigkeitsdruck eingestellt werden.

In einer besonders bevorzugten Ausführungsform wird die Flüssigkeit im Horizontalmischer unterhalb der Produktbettoberfläche der bewegten Polymerpartikelschicht versprüht, vorzugsweise mindestens 10 mm, besonders bevorzugt mindestens 50 mm, ganz besonders bevorzugt mindestens 100 mm, d.h. die Sprühdüse taucht in das Produktbett ein.

Die Produktbettoberfläche ist die Grenzfläche, die sich zwischen den im Horizontalmischer bewegten oberflächennachvernetzten wasserabsorbierenden Polymerpartikeln und der überlagernden Atmosphäre einstellt.

Im Horizontalmischer beträgt der Winkel zwischen der Mischerachse und der Zuführung der Sprühdüse vorzugsweise ca. 90°. Die Flüssigkeit kann senkrecht von oben zugeführt werden. Ein Zuführung schräg von der Seite ist ebenfalls möglich, wobei der Winkel gegenüber der Senkrechten vorzugsweise zwischen 60 und 90°, besonders bevorzugt zwischen 70 und 85°, ganz besonders bevorzugt zwischen 75 und 82,5°, liegt. Die schräge Anordnung der Zuführung ermöglicht die Verwendung kürzerer Zuführungen und damit geringere mechanische Belastungen während des Betriebs des Horizontalmischers.

In einer besonders bevorzugten Ausführungsform befindet sich die Sprühdüse im Horizontalmischer unterhalb der Rotationsachse und sprüht in Rotationsrichtung. Durch diese Anordnung werden die nachbefeuchteten wasserabsorbierenden Polymerpartikel optimal von der Sprühdüse weggefördert. In Kombination mit der schrägen Anordnung ist es auch möglich die Sprühdüse während des Betriebs des Mischers auszutauschen, ohne dass Produkt austritt.

In einer weiteren bevorzugten Ausführungsform der vorliegenden Erfindung wird mindestens eine Sprühdüse thermisch isoliert und/oder begleitbeheizt.

Thermisch isoliert bedeutet, dass die äußere Oberfläche der Sprühdüse zumindest teilweise eine weitere Materialschicht aufweist, wobei das Material der weiteren Materialschicht eine geringere Wärmeleitfähigkeit aufweist als das Material der Sprühdüse. Die Wärmeleitfähigkeit des Materials der weiteren Materialschicht bei 20°C beträgt vorzugsweise weniger als 2 Wm$^{-1}$K$^{-1}$, besonders bevorzugt weniger als 0,5 Wm$^{-1}$K$^{-1}$, ganz besonders bevorzugt weniger als 0,1 Wm$^{-1}$K$^{-1}$.

Begleitbeheizt bedeutet, dass der Sprühdüse zusätzlich Wärmeenergie zugeführt wird, beispielsweise mittels elektrischer Energie oder mittels eines von einem Wärmeträger durchströmten Heizmantels. Geeignete Wärmeträger sind handelsübliche Wärmeträgeröle, wie Marlotherm®, Dampf oder Warmwasser.

Eine mögliche Wärmezufuhr über einen der beim Mischen eingesetzten Einsatzstoffe, d.h. oberflächennachvernetzte wasserabsorbierende Polymerpartikel oder zu versprühende Flüssigkeit, ist keine Begleitbeheizung im Sinne der vorliegenden Erfindung.

Die Temperatur der Sprühdüse ist vorzugsweise von 1 bis 20°C, besonders bevorzugt von 2 bis 15°C, ganz besonders bevorzugt von 5 bis 10°C, höher als die Temperatur der oberflächennachvernetzten wasserabsorbierenden Polymerpartikel.

Im Falle einer thermisch isolierten Sprühdüse ist die Temperatur der zu versprühenden Flüssigkeit vorzugsweise von 1 bis 20°C, besonders bevorzugt von 2 bis 15°C, ganz besonders bevorzugt von 5 bis 10°C, höher als die Temperatur der oberflächennachvernetzten wasserabsorbierenden Polymerpartikel. Die Temperatur der zu versprühenden Flüssigkeit entspricht ungefähr der Temperatur der Sprühdüse.

Im Falle einer begleitbeheizten und optional thermisch isolierten Sprühdüse beträgt die Temperaturdifferenz zwischen den oberflächennachvernetzten wasserabsorbierenden Polymerpartikeln und der aufzusprühenden Flüssigkeit vorzugsweise weniger als 20°C, bevorzugt weniger als 10°C, besonders bevorzugt weniger als 5°C, ganz bevorzugt weniger als 2°C.

Die Temperaturdifferenz zwischen der aufzusprühenden Flüssigkeit und dem Zerstäubergas beträgt vorzugsweise weniger als 20°C, bevorzugt weniger als 10°C, besonders bevorzugt weniger als 5°C, ganz bevorzugt weniger als 2°C.

Die zur Nachbefeuchtung i) einsetzbaren wässrigen Flüssigkeiten enthalten vorzugsweise anorganische partikuläre Substanzen, anorganische kolloidal gelöste Substanzen, organische Polymere, kationische Polymere und/oder polyvalente Metallkationen. Geeignete kationische Polymere und/oder polyvalente Metallkationen liegen in einer bevorzugten Ausführungsform vor der Nachbefeuchtung i) in Form ihrer wasserlöslichen Salze mit organischen oder anorganischen Anionen vor und werden als wässerige Lösungen oder wässrige Dispersionen eingesetzt.

Geeignete anorganische partikuläre Substanzen (anorganische Partikel) sind Tonminerale, wie Montmorillonit, Kaolinit und Talk, wasserunlösliche Sulfate, wie Strontiumsulfat, Kalziumsulfat und Bariumsulfat, Karbonate, wie Magnesiumkarbonat, Kaliumkarbonat und Kalziumkarbonat, Salze polyvalenter Metallkationen, wie Aluminiumsulfat, Aluminiumnitrat, Aluminiumchlorid, Kaliumaluminiumsulfat (Kaliumalaun) und Natriumaluminiumsulfat (Natriumalaun), Magnesiumsulfat, Magnesiumcitrat, Magnesiumlaktat, Zirkoniumsulfat, Zirkoniumlaktat, Eisenlaktat, Eisencitrat, Kalziumazetat, Kalziumpropionat, Kalziumcitrat, Kalziumlaktat, Strontiumlaktat, Zinklaktat, Zinksulfat, Zinkcitrat, Aluminiumlaktat, Aluminiumazetat, Aluminiumformiat, Kalziumformiat, Strontiumformiat, Strontiumazetat, Oxide, wie Magnesiumoxid, Aluminiumoxid, Zinkoxid, Eisen-(II)-oxid, Zirkoniumdioxid und Titandioxid, wasserunlösliche Phosphate, wie Magnesiumphosphat, Strontiumphosphat, Aluminiumphosphat, Eisenphosphat, Zirkoniumphophat und Kalziumphosphat, Diatomeenerde, Polykieselsäuren, Zeolithe und Aktivkohlen. Bevorzugt gelangen Polykieselsäuren zum Einsatz, die je nach Herstellungsart zwischen Fällungskieselsäuren und pyrogenen Kieselsäuren unterschieden werden. Beide Varianten sind unter den Namen Silica FK, Sipernat®, Wessalon® (Fällungskieselsäuren) bzw. Aerosil® (pyrogene Kieselsäuren) kommerziell erhältlich. Vorteilhaft sind auch kolloidale Kieselsäurelösungen, worin die Kieselsäureteilchen typischerweise weniger als 1 $\mu$m Durchmesser aufweisen. Derartige Lösungen sind unter dem Namen Levasil® erhältlich.

Vorzugsweise werden aber wasserunlösliche anorganische Partikel verwendet, beispielsweise pyrogene Kieselsäure, Fällungskieselsäure und wasserunlösliche Metallphosphate. Geeignete wasserunlösliche anorganische Partikel werden in der DE 102 39 074 A1 und geeignete wasserunlösliche Metallphosphate werden in der US 6,831,122 beschrieben, welche beide ausdrücklich Bestandteil der vorliegenden Offenbarung sind.

Wasserunlöslich bedeutet hierbei eine Löslichkeit in Wasser bei 23°C von weniger als 1 g/100 g Wasser, vorzugsweise von weniger als 0,5 g/100 g Wasser, besonders bevorzugt von weniger als 0,1 g/100 g Wasser, ganz besonders bevorzugt von weniger als 0,05 g/100 g Wasser.

Vorzugsweise werden pyrogene Kieselsäuren und/oder Fällungskieselsäuren als anorganische Partikel eingesetzt.

Die anorganischen Partikel weisen eine mittlere Partikelgröße von vorzugsweise weniger als 400 $\mu$m, besonders bevorzugt weniger als 100 $\mu$m, ganz besonders bevorzugt weniger als 50 $\mu$m, auf.
Werden anorganische Partikel eingesetzt, so beträgt die Einsatzmenge bezogen auf die wasserabsorbierenden Polymerpartikel vorzugsweise von 0,05 bis 5 Gew.-%, besonders bevorzugt von 0,1 bis 1,5 Gew.-%, ganz besonders bevorzugt von 0,3 bis 1 Gew.-%.
Geeignete organische Polymere sind alle multifunktionellen Amine mit primären oder sekundären Aminogruppen, wie Polyethylenimin, Polyallylamin und Polylysin. Die gemäß dem erfindungsgemäßen Verfahren bevorzugten organischen Polymere sind Polyamine, wie Polyvinylamin. Besonders geeignete organische Polymere sind in der DE 102 39 074 A1 beschriebenen N-haltigen Polymere, welche ausdrücklich Bestandteil der vorliegenden Offenbarung sind. In einer bevorzugten Ausführungsform werden die dort beschriebenen teilhydrolysierten Poly-N-Vinylcarbonsäureamide eingesetzt. Werden organische Polymere eingesetzt, so beträgt die Einsatzmenge an organischem Polymer bezogen auf die wasserabsorbierenden Polymerpartikel vorzugsweise von 0,1 bis 15 Gew.-%, besonders bevorzugt von 0,5 bis 10 Gew.-%, ganz besonders bevorzugt von 1 bis 5 Gew.-%.
Geeignete kationische Polymere sind kationische Derivate von Polyacrylamiden und polyquartäre Amine. Die Anionen der eingesetzten kationischen Polymere sind alle bekannten organischen und anorganischen Anionen, bevorzugt sind Chlorid, Formiat, Azetat, Propionat, Malat, Tartrat, Citrat und Laktat. Die kationischen Polymere können aber beispielsweise auch als Sulfate, Phosphate oder Karbonate eingesetzt werden, wobei gegebenenfalls schwer wasserlösliche Salze entstehen, die als Pulver oder wässrige Dispersionen eingesetzt werden können.

Polyquartäre Amine sind beispielsweise Kondensationsprodukte aus Hexamethylendiamin, Dimethylamin und Epichlorhydrin, Kondensationsprodukte aus Dimethylamin und Epichlorhydrin, Copolymere aus Hydroxyethylcellulose und Diallyldimethylammoniumchlorid, Copolymere aus Acrylamid und $\alpha$-Methacrylyloxyethyltrimethylammoniumchlorid, Kondensationsprodukte aus Hydroxyethylcellulose, Epichlorhydrin und Trimethylamin, Homopolymerisate von Diallyldimethylammoniumchlorid und Additionsprodukte von Epichlorhydrin an Amidoamine. Des Weiteren können polyquartäre Amine durch Umsetzung von Dimethylsulfat mit Polymeren, wie Polyethyleniminen, Copolymere aus Vinylpyrrolidon und Dimethylaminoethylmethacrylat oder Copolymere aus Ethylmethacrylat und Diethylaminoethylmethacrylat erhalten werden. Die polyquartären Amine sind in einem breiten Molekulargewichtsbereich verfügbar.

Werden kationische Polymere eingesetzt, so beträgt die Einsatzmenge an kationischem Polymer bezogen auf die wasserabsorbierenden Polymerpartikel vorzugsweise von 0,1 bis 15 Gew.-%, besonders bevorzugt von 0,5 bis 10 Gew.-%, ganz besonders bevorzugt von 1 bis 5 Gew.-%.

Geeignete polyvalente Metallkationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Karbonat, Hydrogenkarbonat, Nitrat, Phosphat, Hydrogenphosphat, Dihydrogenphosphat und Carboxylat, wie Azetat, Citrat, Tartrat und Laktat, möglich. Aluminiumsulfat, Aluminiumlaktat und Zirkoniumsulfat sind bevorzugt. Bei Vorliegen verschiedener Diastereomere wie im Falle der Weinsäure sind alle Formen eingeschlossen und können als Anionen für alle erfindungsgemäß einsetzbaren polyvalenten Metallkationen verwendet werden. Die polyvalenten Metallkationen werden vorzugsweise als Lösung eingesetzt.

Weitere besonders geeignete polyvalente Metallkationen sind in WO 2005/080479 A1 beschrieben, welche ausdrücklich Bestandteil der vorliegenden Offenbarung sind. Es können weiterhin beliebige Gemische der löslichen Salze ein- und polyvalenter Metallkationen eingesetzt werden, beispielsweise kann eine geeignete wässrige Lösung durch Auflösen von Milchsäure oder Alkalimetalllaktat gemeinsam mit Aluminiumsulfat hergestellt und eingesetzt werden. Das Prinzip kann auf beliebige Salze polyvalenter Metallkationen verallgemeinert werden. Es können auch Gemische von verschiedenen polyvalenten Metallkationen oder beliebige Gemische von deren Salzen eingesetzt werden, wie beispielsweise Zirkoniumlaktat und Aluminiumlaktat, Aluminiumlaktat und Kalziumlaktat, Zirkoniumlaktat und Kalziumcitrat.

Des Weiteren können beliebige organische und anorganische Salze einwertiger Kationen, bevorzugt Alkalimetallsalze, organische Säuren und/oder anorganische Säuren zusätzlich in der Lösung mit den polyvalenten Metallkationen anwesend sein. Beispiele hierfür sind Alkalimetallphosphate, Alkalimetallsulfate, Alkalimetallhydrogensulfate, Alkalimetalldihydrogenphosphate, Alkalimetallhydrogenkarbonate, Alkalimetallhydrogensulfite, sowie Formiate, Azetate, Laktate, Propionate, Tartrate, Citrate, Malate der Alkalimetalle, des Ammoniums und des Triethanolamoniums.

Werden polyvalente Metallkationen eingesetzt, so beträgt die Einsatzmenge an polyvalenten Metallkationen bezogen auf die wasserabsorbierenden Polymerpartikel üblicherweise mindestens 0,0001 Gew.%, vorzugsweise von 0,005 bis 5 Gew.-%, besonders bevorzugt von 0,05 bis 1,5 Gew.-%, ganz besonders bevorzugt von 0,1 bis 1 Gew.-%.

Die nachbefeuchteten Polymerpartikel werden anschließend pneumatisch gefördert.

Grundsätzlich lassen sich bei der pneumatischen Förderung drei verschiedene Förderarten unterscheiden.

- Bei Flugförderung und Strömförderung im Bereich hoher Gasgeschwindigkeiten gelten annähernd die Gesetze des frei angeströmten Einzelkorns. Es ist die klassische Art der pneumatischen Förderung. Es treten keinerlei Produktablagerungen auf. Es liegt eine im Wesentlichen gleichmäßige Fördergutverteilung im Rohr vor.

- Sinkt die Gasgeschwindigkeit, dann kommt man in den Bereich der Strähnenförderung, wo das Fördergut vor allem in der unteren Rohrhälfte strömt. In der oberen Rohrhälfte liegt Flugförderung vor.

- Bei kleinen Gasgeschwindigkeiten erfolgt die Förderung äußerst schonend als Dichtstromförderung (Pfropfenförderung, Impulsförderung) mit hohem Druckverlust.

Prinzipiell kann die Druckförderung mit langsameren Fördergeschwindigkeiten arbeiten als die Saugförderung, da im Überdruck die Druckreserven größer sind als im Vakuum, und da mit steigendem Druck die Fördergasdichte, die das Produkt vorantreibt, zunimmt.

Da Fördergas kompressibel ist, herrscht in der Förderleitung kein konstanter Druck, sondern am Anfang ein höherer als am Ende. Dadurch verändert sich aber auch das Gasvolumen, so dass am Anfang, bei höherem Druck, langsamere Gasgeschwindigkeiten vorherrschen und am Ende, bei niedrigerem Druck, höhere Gasgeschwindigkeiten.

H. Kalman, Powder Technology 104 (1999) 214 bis 220 beschreibt Untersuchungen zum Abrieb in pneumatischen Fördersystemen. Aufgrund der geringeren mechanischen Belastung sind niedrigere Fördergeschwindigkeiten vorteilhaft. Gemäß der Publikation werden bei der pneumatischen Förderung aber oft aus Sicherheitsgründen oft unnötig hohe Fördergeschwindigkeiten gewählt.

Zu niedrige Fördergeschwindigkeiten im Bereich der Strähnenförderung sind problematisch, da in dem instabilen Bereich zwischen der Dichtstromförderung und der Strähnenförderung keine stabile Förderung möglich ist. Vielmehr können die dabei auftretenden mechanischen Belastungen zu schweren Schäden am Fördersystem, bis zum Herausreißen der Förderleitungen aus den Halterungen, führen.

Die optimale Gasanfangsgeschwindigkeit bei der pneumatischen Förderung ii) hängt vom Durchmesser der Förderleitung ab. Diese Abhängigkeit wird am besten mit der Froude-Zahl beschrieben. Für die pneumatische Förderung ist die Froude-Zahl wie folgt definiert:

$$Fr = \frac{v}{\sqrt{D \times g}}$$

v    Gasgeschwindigkeit
D    Innendurchmesser der Transportleitung
g    Erdbeschleunigung

Die Froude-Zahl bei der pneumatischen Förderung ii) wasserabsorbierender Polymerpartikel beträgt vorzugsweise von 10 bis 18, besonders bevorzugt von 11 bis 16, ganz besonders bevorzugt von 12 bis 14.

Bei zu niedrigen Fördergeschwindigkeiten wird die pneumatische Förderung ii) instabil und höhere Fördergeschwindigkeiten erhöhen den unerwünschten Abrieb infolge steigender mechanischer Belastung.

6

Die Fördergutbeladung der pneumatischen Förderung ii) beträgt vorzugsweise von 0,5 bis 20 kg/kg, besonders bevorzugt von 1 bis 10 kg/kg, ganz besonders bevorzugt von 2 bis 6 kg/kg, wobei die Fördergutbeladung der Quotient aus Fördergutmassenstrom und Gasmassenstrom ist.

Grundsätzlich erhöht sich mit steigender Fördergutbeladung auch die optimale Gasanfangsgeschwindigkeit.

Der Durchmesser der Rohrleitung, in der die pneumatische Förderung ii) durchgeführt wird, beträt vorzugsweise von 3 bis 30 cm, besonders bevorzugt von 4 bis 25 cm, ganz besonders bevorzugt von 5 bis 20 cm. Zu niedrige Rohrdurchmesser führen zu einer höheren mechanischen Belastung durch die pneumatische Förderung ii) und begünstigen damit den unerwünschten Abrieb. Zu große Rohrdurchmesser ermöglichen ein ebenso unerwünschtes Absetzen der wasserabsorbierenden Polymerpartikel in der Förderleitung.

Um die mechanische Belastung gering zu halten sollte die Anzahl der Bögen in der Rohrleitung eines pneumatischen Fördersystems möglichst gering sein, vorzugsweise weniger als 6, bevorzugt weniger als 5, besonders bevorzugt weniger als 4, ganz besonders bevorzugt weniger als 3. Eine Rohrleitung in einem pneumatischen Fördersystem ist der Abschnitt zwischen der Aufgabeeinrichtung für die wasserabsorbierenden Polymerpartikel und dem Empfangsbehälter, d.h. der Bereich in dem die wasserabsorbierenden Polymerpartikel im Gasstrom transportiert werden.

Der zeitliche Abstand (Verweilzeit) zwischen der Nachbefeuchtung i) und der pneumatischen Förderung ii) beträgt vorzugsweise weniger als 45 Minuten, besonders bevorzugt weniger als 30 Minuten, ganz besonders bevorzugt weniger als 15 Minuten.

Der vorliegenden Erfindung liegt die Erkenntnis zugrunde, dass das bei der Nachbefeuchtung eingesetzte Wasser die Elastizität der wasserabsorbierenden Polymerpartikel erhöht. Für die Elastizität der wasserabsorbierenden Polymerpartikel scheint aber nur das Wasser nahe der Partikeloberfläche wichtig zu sein. An der Partikeloberfläche ist die Wasserkonzentration aber kurz nach der Nachbefeuchtung am größten. Mit der Zeit diffundiert das Wasser langsam von der Partikeloberfläche in das Partikelinnere und die Wasserkonzentration an der Partikeloberfläche fällt wieder.

Bei der pneumatischen Förderung werden die wasserabsorbierenden Polymerpartikel mechanisch belastet. Elastische wasserabsorbierende Polymerpartikel werden weniger stark geschädigt. Somit ist es vorteilhaft wasserabsorbierende Polymerpartikel unmittelbar nach der Nachbefeuchtung pneumatisch zu fördern.

Anschließend können die nachbefeuchteten Polymerpartikel klassiert werden, wobei zu kleine und/oder zu große Polymerpartikel abgetrennt und in das Verfahren rückgeführt werden.

Die für die Klassierung iii) geeigneten Siebmaschinen unterliegen keiner Beschränkung. Vorzugsweise werden Taumelsiebmaschinen eingesetzt. Geeignete Taumelsiebmaschinen sind beispielsweise bei ALLGAIER Werke GmbH, Uhingen, DE, und MINOX Siebtechnik GmbH, Offenbach/Queich, DE, erhältlich.

Bei einer Taumelsiebmaschine werden die zu klassierenden wasserabsorbierenden Polymerpartikel infolge einer erzwungenen Vibration spiralförmig über das Sieb bewegt. Der relativ lange Siebweg bei kleiner Siebfläche führt zu einer hohen Trennschärfe bei der Klassierung. Die erzwungene Vibration hat typischerweise eine Amplitude von 0,7 bis 40 mm, vorzugsweise von 1,5 bis 25 mm, und eine Frequenz von 1 bis 100 Hz, vorzugsweise von 5 bis 10 Hz.

Die Taumelsiebmaschinen weisen vorzugsweise mindestens 2, besonders bevorzugt mindestens 3, ganz besonders bevorzugt mindestens 4, Siebe auf. Vorteilhaft werden dabei die vom oberen Sieb herabfallenden wasserabsorbierenden Polymerpartikel durch eine, vorzugsweise trichterförmige, Vorrichtung in Richtung Siebmitte des unteren Siebes abgelenkt.

Die Maschenweite der Siebe liegt vorzugsweise im Bereich von 100 bis 1.000 $\mu$m, besonders bevorzugt im Bereich von 150 bis 850 $\mu$m, ganz besonders bevorzugt im Bereich von 150 bis 600 $\mu$m.

Die wasserabsorbierenden Polymerpartikel weisen während des Klassierens vorzugsweise eine Temperatur von 40 bis 120°C, besonders bevorzugt von 45 bis 100°C, ganz besonders bevorzugt von 50 bis 80°C, auf.

Besonders vorteilhaft wird die Klassierung kontinuierlich durchgeführt. Der Durchsatz an wasserabsorbierenden Polymerpartikeln beträgt dabei üblicherweise mindestens 100 kg/m$^2$·h, vorzugsweise mindestens 150 kg/m$^2$·h, bevorzugt mindestens 200 kg/m$^2$·h, besonders bevorzugt mindestens 250 kg/m$^2$·h, ganz besonders bevorzugt mindestens 300 kg/m$^2$·h.

Vorzugsweise werden die wasserabsorbierenden Polymerpartikel während des Klassierens mit einem Gasstrom, besonders bevorzugt Luft, überströmt. Die Gasmenge beträgt typischerweise von 0,1 bis 10 m$^3$/h pro m$^2$ Siebfläche, vorzugsweise von 0,5 bis 5 m$^3$/h pro m$^2$ Siebfläche, besonders bevorzugt von 1 bis 3 m$^3$/h pro m$^2$ Siebfläche, wobei das Gasvolumen unter Standardbedingungen gemessen wird (25°C und 1 bar). Besonders bevorzugt wird der Gasstrom vor dem Eintritt in die Siebvorrichtung angewärmt, typischerweise auf eine Temperatur von 40 bis 120°C, vorzugsweise auf eine Temperatur von 50 bis 110°C, bevorzugt auf eine Temperatur von 60 bis 100°C, besonders bevorzugt auf eine Temperatur von 65 bis 90°C, ganz besonders bevorzugt auf eine Temperatur von 70 bis 80°C. Der Wassergehalt des Gasstroms beträgt typischerweise weniger 5 g/kg, vorzugsweise weniger als 4,5 g/kg, bevorzugt weniger als 4 g/kg, besonders bevorzugt weniger als 3,5 g/kg, ganz besonders bevorzugt weniger als 3 g/kg. Ein Gasstrom mit geringem Wassergehalt kann beispielsweise erzeugt werden, indem aus einem Gasstrom mit höherem Wassergehalt eine entsprechende Wassermenge durch Abkühlung auskondensiert wird.

In einer bevorzugten Ausführungsform der vorliegenden Erfindung werden mehrere Taumelsiebmaschinen parallel betrieben.

Die Taumelsiebmaschinen werden üblicherweise elektrisch geerdet.

Der zeitliche Abstand (Verweilzeit) zwischen der Nachbefeuchtung i) und der Klassierung iii) beträgt vorzugsweise mindestens 30 Minuten, besonders bevorzugt mindestens 45 Minuten, ganz besonders bevorzugt mindestens 60 Minuten.

Der vorliegenden Erfindung liegt die Erkenntnis zugrunde, dass das bei der Nachbefeuchtung eingesetzte Wasser die Glasübergangstemperatur der wasserabsorbierenden Polymerpartikel senkt, die Partikeloberfläche wird klebrig. An der Partikeloberfläche ist die Wasserkonzentration aber kurz nach der Nachbefeuchtung am größten. Mit der Zeit diffundiert das Wasser langsam von der Partikeloberfläche in das Partikelinnere und die Wasserkonzentration an der Partikeloberfläche fällt wieder. Somit durchläuft die Klebrigkeit der Partikeloberfläche ein Maximum.

Die Klebrigkeit der Partikeloberfläche führt aber dazu, dass sehr kleine Partikel fest an größeren Partikeln anhaften, wodurch diese anklebenden sehr kleinen Partikel nur schwer durch Klassierung abgetrennt werden können. Daher ist es vorteilhaft nach der Nachbefeuchtung noch eine ausreichende Zeit mit der Klassierung zu warten. In dieser Zeit diffundiert ein Teil des Wassers in das Partikeloberfläche und die Klebrigkeit der Partikeloberfläche nimmt wieder ab.

Um die gewünschte Verweilzeit zwischen Nachbefeuchtung i) und Klassierung iii) zu erreichen, können die wasserabsorbierenden Polymerpartikel in geeigneten Behältern zwischengelagert werden.

Im Folgenden wird die Herstellung der oberflächennachvernetzten Polymerpartikel näher erläutert:

Die wasserabsorbierenden Polymerpartikel werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm$^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit und der in der Monomerlösung enthaltende Polymerisationsinhibitor deaktiviert werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße (Feinkorn) an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 μm, besonders bevorzugt von 250 bis 600 μm, ganz besonders von 300 bis 500 μm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 μm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel (Feinkorn) niedrig sein.

Zu kleine Polymerpartikel werden daher üblicherweise abgetrennt und in das Verfahren rückgeführt. Die geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 μm, beträgt vorzugsweise mindestens 90 Gew.-%, besonders bevorzugt mindesten 95 Gew.-%, ganz besonders bevorzugt mindestens 98 Gew.-%.

Vorteilhaft beträgt der Anteil an Partikeln mit einer Partikelgröße von höchstens 600 μm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel werden zur weiteren Verbesserung der Eigenschaften oberflächennachvernetzt. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Glyzerin, Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Karbonat, Hydrogenkarbonat, Nitrat, Phosphat, Hydrogenphosphat, Dihydrogenphosphat und Carboxylat, wie Acetat, Citrat und Laktat, möglich. Aluminiumsulfat und Aluminumlaktat sind bevorzugt. Außer Metallsalzen können auch polyfunktionelle Amine als polyvalente Kationen eingesetzt werden. Polyfunktionelle Amine sind Verbindungen mit mindestens zwei Amino- und/oder Ammonium-Gruppen. Vorzugsweise werden aber Metallkationen als polyvalenten Kationen eingesetzt.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Die gemäß dem erfindungsgemäßen Verfahren hergestellten wasserabsorbierenden Polymerpartikel weisen einen Feuchtegehalt von vorzugsweise 1 bis 15 Gew.-%, besonders bevorzugt 1,5 bis 10 Gew.-%, ganz besonders bevorzugt 2 bis 8 Gew.-%, auf, wobei der Wassergehalt gemäß der von der EDANA empfohlenen Testmethode Nr. WSP 230.2-05 "Moisture Content" bestimmt wird.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Centrifuge Retention Capacity" bestimmt.

Die gemäß dem erfindungsgemäßen Verfahren hergestellten wasserabsorbierenden Polymerpartikel weisen eine Absorption unter einem Druck von 49,2 g/cm$^2$ von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm$^2$ der wasserabsorbierenden Polymerpartikel beträgt üblicherweise weniger als 35 g/g. Die Absorption unter einem Druck von 49,2 g/cm$^2$ wird analog der von der EDANA empfohlenen Testmethode Nr. WSP 242.2-05 "Absorption under Pressure" bestimmt, wobei statt eines Drucks von 21,0 g/cm$^2$ ein Druck von 49,2 g/cm$^2$ eingestellt wird.

Die wasserabsorbierenden Polymerpartikel werden mittels der nachfolgend beschriebenen Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

Flüssigkeitsweiterleitung (Saline Flow Conductivity)

Die Flüssigkeitsweiterleitung (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$SFC \text{ [cm}^3\text{s/g]} = (Fg(t=0) \times L0)/(d \times A \times WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in g/cm$^3$, A die Fläche der Gelschicht in cm$^2$ und WP der hydrostatische Druck über der Gelschicht in dyn/cm$^2$.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Centrifuge Retention Capacity" bestimmt.

Absorption unter einem Druck von 49,2 g/cm$^2$ (Absorption under Pressure)

Die Absorption unter einem Druck von 49,2 g/cm$^2$ (AUL0.7psi) wird analog der von der EDANA (European Disposables and Nonwovens Association) empfohlenen Testmethode Nr. WSP 242.2-05 "Absorption under Pressure" bestimmt, wobei statt eines Drucks von 21,0 g/cm$^2$ (AUL0.3psi) ein Druck von 49,2 g/cm$^2$ (AUL0.7psi) eingestellt wird.
Die EDANA-Testmethoden sind beispielsweise erhältlich bei der EDANA, Avenue Eugene Plasky 157, B-1030 Brüssel, Belgien.

Beispiele

Beispiel 1 (Herstellung der wasserabsorbierenden Polymerpartikel)

Durch kontinuierliches Mischen von entionisiertem Wasser, 50 gew.-%iger Natronlauge und Acrylsäure wurde eine Acrylsäure/Natriumacrylatlösung hergestellt, so dass der Neutralisationsgrad 71,3 mol-% entsprach. Der Feststoffgehalt der Monomerlösung betrug 38,8 Gew.-%.
Als mehrfach ethylenisch ungesättigter Vernetzer wurde Polyethylenglykol-400-diacrylat (Diacrylat ausgehend von einem Polyethylenglykol mit einem mittleren Molgewicht von 400 g/mol) verwendet. Die Einsatzmenge betrug 2 kg Vernetzer pro t Monomerlösung.
Zur Initiierung der radikalischen Polymerisation wurden pro t Monomerlösung 1,03 kg einer 0,25gew.-%igen wässriger Wasserstoffperoxidlösung, 3,10 kg einer 15gew.-%igen wässrigen Natriumperoxodisulfatlösung und 1,05 kg einer 1gew.-%igen wässrigen Ascorbinsäurelösung eingesetzt.
Der Durchsatz der Monomerlösung betrug 20 t/h. Die Reaktionslösung hatte am Zulauf eine Temperatur von 23,5°C.
Die einzelnen Komponenten wurden in folgenden Mengen kontinuierlich in einen Reaktor vom Typ List Contikneter mit einem Volumen 6,3m$^3$ (LIST AG, Arisdorf, CH) dosiert:

| | |
|---|---|
| 20 t/h | Monomerlösung |
| 40 kg/h | Polyethylenglykol-400-diacrylat |
| 82,6 kg/h | Wasserstoffperoxidlösung/Natriumperoxodisulfatlösung |
| 21 kg/h | Ascorbinsäurelösung |

Zwischen dem Zugabepunkt für den Vernetzer und den Zugabestellen für die Initiatoren wurde die Monomerlösung mit Stickstoff inertisiert.
Es fand nach ca. 50% der Verweilzeit zusätzlich eine Zudosierung von aus dem Herstellungsprozeß durch Mahlung und Siebung anfallendem Feinkorn (1000 kg/h) in den Reaktor statt. Die Verweilzeit der Reaktionsmischung im Reaktor betrug 15 Minuten.
Das erhaltene Polymergel wurde auf einen Bandtrockner aufgegeben. Auf dem Bandtrockner wurde das Polymergel kontinuierlich mit einem Luft/Gasgemisch umströmt und getrocknet. Die Verweilzeit im Bandtrockner betrug 37 Minuten.
Das getrocknete Polymergel wurde gemahlen und auf eine Partikelgrößenfraktion von 150 bis 850 μm abgesiebt. Das erhaltene Grundpolymer wurde oberflächennachvernetzt.
In einem Schugi Flexomix® (Hosokawa Micron B.V., Doetinchem, NL) wurde das Grundpolymer mit einer Oberflächennachvernetzerlösung beschichtet und anschließend in einem NARA Paddle Dryer (GMF Gouda, Waddinxveen, NL) 45 Minuten bei 190°C getrocknet.
Es wurden folgende Mengen in den Schugi Flexomix® dosiert:

| | |
|---|---|
| 7,5 t/h | Grundpolymer |
| 270,0 kg/h | Oberflächennachvernetzerlösung |

Die Oberflächennachvernetzerlösung enthielt 2,8 Gew.-% 2-Hydroxyethyl-2 oxazolidon, 2,8 Gew.-% Aluminiumsulfat, 66,1 Gew.-% entionisiertes Wasser und 28,3 Gew.-% Isopropanol.

Nach dem Trocken wurde das oberflächennachvernetzte Grundpolymer in einem NA-RA Paddle-Cooler (GMF Gouda, Waddinxveen, NL) auf ca. 60°C abgekühlt.

Die erhaltenen wasserabsorbierenden Polymerpartikel wiesen eine Zentrifugenretentionskapazität (CRC) von 28,4 g/g auf.

Beispiel 2

In einer Küchenmaschine vom Typ ProfiMixx 47 (Robert Bosch GmbH; Gerlingen-Schillerhöhe; DE) wurden 220 g wasserabsorbierende Polymerpartikel aus Beispiel 1 vorgelegt, unter Rühren (Stufe 4; ca. 500 Upm) mit 5,5 g Wasser nachbefeuchtet und eine Minute nachgerührt. Das Wasser wurde mittels einer Schlauchpumpe mit einer Dosiergeschwindigkeit von 5 g/min aufgesprüht. Der Schlauch hatte einen Innendurchmesser von 2,54 mm.

Jeweils 20 g der nachbefeuchteten wasserabsorbierenden Polymerpartikel wurden nach 0, 2, 4, 6, 8, 10, 30, 60 bzw. 120 Minuten mechanisch belastet. Dazu wurden die wasserabsorbierenden Polymerpartikel zusammen mit 30 Keramikkörpern mit runden Kappen (je ca. 7,4 g) in einer Kugelmühle 5 Minuten bei 150 Upm gemahlen.

Anschließend wurde die Partikelgrößenverteilung gemessen. Die angegebenen Prozentwerte sind Gewichtsprozent. Die Ergebnisse sind in Tabelle 1 zusammengefasst:

Tab. 1: Nachbefeuchtung mit 2,5 Gew.-% Wasser und 5 Minuten Belastungszeit

| Minuten | <150$\mu$m | 150-180$\mu$m | 180-300$\mu$m | 300-600$\mu$m | 600-850$\mu$m | >850$\mu$m | SFC [$10^{-7}$cm$^3$s/g] |
|---|---|---|---|---|---|---|---|
| 0 | 0,8 % | 0,9 % | 12,2 % | 51,5 % | 34,5 % | 0,2 % | 17 |
| 2 | 0,6 % | 1,0 % | 12,2 % | 49,0 % | 36,9 % | 0,2 % | 22 |
| 4 | 0,7 % | 0,8 % | 12,0 % | 50,9 % | 35,3 % | 0,2 % | 29 |
| 6 | 0,8 % | 0,8 % | 11,2 % | 48,9 % | 37,6 % | 0,8 % | 25 |
| 8 | 1,0 % | 1,0 % | 13,4 % | 49,0 % | 35,5 % | 0,2 % | 22 |
| 10 | 1,2 % | 1,1 % | 13,6 % | 49,6 % | 34,5 % | 0,2 % | 22 |
| 30 | 1,4 % | 1,2 % | 13,8 % | 48,9 % | 34,8 % | 0,2 % | 20 |
| 60 | 1,4 % | 1,2 % | 13,8 % | 48,8 % | 34,7 % | 0,1 % | 22 |
| 120 | 1,5 % | 1,4 % | 13,7 % | 48,5 % | 35,0 % | 0,1 % | 23 |

Beispiel 3

Es wurde verfahren wie unter Beispiel 2. Statt 5 Minuten mit 30 Keramikkörpern wurde 15 Minuten mit 40 Keramikkörpern gemahlen.

Anschließend wurde die Partikelgrößenverteilung gemessen. Die angegebenen Prozentwerte sind Gewichtsprozent. Die Ergebnisse sind in Tabelle 2 zusammengefasst:

Tab. 2: Nachbefeuchtung mit 2,5 Gew.-% Wasser und 15 Minuten Belastung

| Minuten | <150$\mu$m | 150-180$\mu$m | 180-300$\mu$m | 300-600$\mu$m | 600-850$\mu$m | >850$\mu$m |
|---|---|---|---|---|---|---|
| 0 | 1,1 % | 0,1 % | 12,6 % | 64,8 % | 21,6 % | 0,0 % |
| 2 | 2,4 % | 1,5 % | 16,5 % | 58,2 % | 21,7 % | 0,0 % |
| 4 | 3,4 % | 1,9 % | 17,2 % | 56,9 % | 20,5 % | 0,1 % |
| 6 | 4,7 % | 2,3 % | 18,8 % | 56,3 % | 18,0 % | 0,0 % |
| 8 | 6,0 % | 2,6 % | 18,7 % | 54,9 % | 17,8 % | 0,0 % |
| 10 | 5,7 % | 2,7 % | 21,4 % | 56,2 % | 14,0 % | 0,0 % |
| 30 | 5,5 % | 2,7 % | 19,8 % | 55,5 % | 16,5 % | 0,0 % |
| 60 | 7,1 % | 2,7 % | 21,6 % | 55,1 % | 13,8 % | 0,0 % |
| 120 | 8,8 % | 3,2 % | 21,6 % | 55,6% | 10,7 % | 0,0 % |

Beispiel 4

Es wurde verfahren wie unter Beispiel 3. Statt mit 5,5 g Wasser wurde mit 11,0 g Wasser nachbefeuchtet.

Anschließend wurde die Partikelgrößenverteilung gemessen. Die angegebenen Prozentwerte sind Gewichtsprozent. Die Ergebnisse sind in Tabelle 3 zusammengefasst:

Tab. 3: Nachbefeuchtung mit 5 Gew.-% Wasser und 15 Minuten Belastung

| Minuten | <150$\mu$m | 150-180$\mu$m | 180-300$\mu$m | 300-600$\mu$m | 600-850$\mu$m | >850$\mu$m |
|---|---|---|---|---|---|---|
| 0 | 0,6 % | 0,8 % | 12,1 % | 55,8 % | 30,7 % | 0,0 % |
| 2 | 0,3 % | 0,5 % | 8,6 % | 51,4 % | 39,0 % | 0,3 % |
| 4 | 0,5 % | 0,5 % | 9,0 % | 52,5 % | 37,4 % | 0,1 % |
| 6 | 1,0 % | 0,9 % | 13,7 % | 56,9 % | 27,7 % | 0,0 % |
| 8 | 0,9 % | 0,9 % | 11,8 % | 52,2 % | 34,3 % | 0,0 % |
| 10 | 0,7 % | 0,8 % | 11,6 % | 52,7 % | 34,1 % | 0,1 % |
| 30 | 0,8 % | 1,0 % | 13,7 % | 60,3 % | 24,4 % | 0,0 % |
| 60 | 1,2 % | 1,3 % | 15,0 % | 59,1 % | 23,3 % | 0,1 % |
| 120 | 1,6 % | 1,4 % | 15,7 % | 56,8 % | 24,7 % | 0,0 % |

In den Tabellen 1 bis 3 ist jeweils ein deutlicher Anstieg kleiner Polymerpartikel (<150$\mu$m) mit zunehmender Verweilzeit zwischen Nachbefeuchtung und mechanischer Belastung erkennbar.

**Patentansprüche**

1. Verfahren zur Herstellung wasserabsorbierender Polymerpartikel durch Polymerisation einer Monomerlösung oder -suspension, enthaltend

    a) mindestens ein ethylenisch ungesättigtes, säuregruppentragendes Monomer, das zumindest teilweise neutralisiert sein kann,
    b) mindestens einen Vernetzer,
    c) mindestens einen Initiator,
    d) optional ein oder mehrere mit den unter a) genannten Monomeren copolymerisierbare ethylenisch ungesättigte Monomere und
    e) optional ein oder mehrere wasserlösliche Polymere,

    umfassend Trocknung, Mahlung, Klassierung, und Oberflächennachvernetzung, wobei

    i) die oberflächennachvernetzten Polymerpartikel nachbefeuchtet,
    ii) die nachbefeuchteten Polymerpartikel pneumatisch gefördert werden und
    iii) optional die nachbefeuchteten Polymerpartikel klassiert werden,

    **dadurch gekennzeichnet, dass** zur Nachbefeuchtung i) ein Mischer mit rotierenden Mischwerkzeugen eingesetzt wird und der zeitliche Abstand der Nachbefeuchtung i) und der pneumatischen Förderung ii) weniger als eine Stunde beträgt.

2. Verfahren gemäß Anspruch 1, **dadurch gekennzeichnet, dass** die wasserabsorbierenden Polymerpartikel durch Ausbildung kovalenter Bindungen oberflächennachvernetzt werden.

3. Verfahren gemäß Anspruch 2, **dadurch gekennzeichnet, dass** bei der Oberflächennachvernetzung zusätzlich polyvalente Metallkationen eingesetzt werden.

4. Verfahren gemäß einem der Ansprüche 1 bis 3, **dadurch gekennzeichnet, dass** die der Nachbefeuchtung i) zugeführten wasserabsorbierenden Polymerpartikel eine Temperatur von 40 bis 80°C aufweisen.

**5.** Verfahren gemäß einem der Ansprüche 1 bis 4, **dadurch gekennzeichnet, dass** zur Nachbefeuchtung i) eine wässrige Lösung oder eine wässrige Dispersion, enthaltend eine anorganische partikuläre Substanz, eine anorganische kolloidal gelöste Substanz, ein organisches Polymer, ein kationisches Polymer und/oder ein Salz eines polyvalenten Metallkations, eingesetzt wird.

**6.** Verfahren gemäß einem der Ansprüche 1 bis 5, **dadurch gekennzeichnet, dass** die der pneumatischen Förderung ii) zugeführten wasserabsorbierenden Polymerpartikel eine Temperatur von 40 bis 80°C aufweisen.

**7.** Verfahren gemäß einem der Ansprüche 1 bis 6, **dadurch gekennzeichnet, dass** die Anfangsgeschwindigkeit bei der pneumatischen Förderung ii) einer Froude-Zahl von 10 bis 18 entspricht.

**8.** Verfahren gemäß einem der Ansprüche 1 bis 7, **dadurch gekennzeichnet, dass** mindestens 95 Gew.-% der wasserabsorbierenden Polymerpartikel eine Partikelgröße von mindestens 150 $\mu$m aufweisen.

**9.** Verfahren gemäß einem der Ansprüche 1 bis 8, **dadurch gekennzeichnet, dass** mindestens 95 Gew.-% der wasserabsorbierenden Polymerpartikel eine Partikelgröße von höchstens 600 $\mu$m aufweisen.

**10.** Verfahren gemäß einem der Ansprüche 1 bis 9, **dadurch gekennzeichnet, dass** die wasserabsorbierenden Polymerpartikel eine Zentrifugenretentionskapazität von mindestens 15 g/g aufweisen.

## Claims

**1.** A process for producing water-absorbing polymer particles by polymerizing a monomer solution or suspension comprising

a) at least one ethylenically unsaturated monomer which bears acid groups and may be at least partly neutralized,
b) at least one crosslinker,
c) at least one initiator,
d) optionally one or more ethylenically unsaturated monomers copolymerizable with the monomers mentioned under a) and
e) optionally one or more water-soluble polymers,

comprising drying, grinding, classifying and surface postcrosslinking, in which

i) the surface postcrosslinked polymer particles are remoisturized,
ii) the remoisturized polymer particles are pneumatically delivered, and
iii) optionally, the remoisturized polymer particles are classified,

wherein a mixer with rotating mixing tools is used for the remoisturization i) and the time between the remoisturization i) and the pneumatic delivery ii) is less than one hour.

**2.** The process according to claim 1, wherein the water-absorbing polymer particles are surface postcrosslinked by formation of covalent bonds.

**3.** The process according to claim 2, wherein polyvalent metal cations have been used additionally in the surface postcrosslinking.

**4.** The process according to any of claims 1 to 3, wherein the water-absorbing polymer particles supplied to the remoisturization i) have a temperature of 40 to 80°C.

**5.** The process according to any of claims 1 to 4, wherein remoisturization i) is effected using an aqueous solution or an aqueous dispersion comprising an inorganic particulate substance, an inorganic colloidally dissolved substance, an organic polymer, a cationic polymer and/or a salt of a polyvalent metal cation.

**6.** The process according to any of claims 1 to 5, wherein the water-absorbing polymer particles supplied to the pneumatic delivery ii) have a temperature of 40 to 80°C.

**7.** The process according to any of claims 1 to 6, wherein the initial velocity in the pneumatic delivery ii) corresponds to a Froude number of from 10 to 18.

**8.** The process according to any of claims 1 to 7, wherein at least 95% by weight of the water-absorbing polymer particles have a particle size of at least 150 $\mu$m.

**9.** The process according to any of claims 1 to 8, wherein at least 95% by weight of the water-absorbing polymer particles have a particle size of at most 600 $\mu$m.

**10.** The process according to any of claims 1 to 9, wherein the water-absorbing polymer particles have a centrifuge retention capacity of at least 15 g/g.

**Revendications**

**1.** Procédé pour la préparation de particules polymères absorbant l'eau par polymérisation d'une solution ou d'une suspension de monomères, contenant

a) au moins un monomère éthyléniquement insaturé, portant des groupes acides, qui peut être au moins partiellement neutralisé,
b) au moins un réticulant,
c) au moins un initiateur,
d) éventuellement un ou plusieurs monomères éthyléniquement insaturés copolymérisables avec les monomères cités en a) et
e) éventuellement un ou plusieurs polymères solubles dans l'eau,

comprenant le séchage, le broyage, la classification et la postréticulation en surface,

i) les particules polymères postréticulées en surface étant posthumidifiées,
ii) les particules polymères posthumidifiées étant transportées pneumatiquement et
iii) les particules polymères posthumidifiées étant éventuellement classées,

**caractérisé en ce qu'**on utilise, pour la posthumidification i), un mélangeur présentant des outils de mélange rotatifs et l'intervalle temporel entre la posthumidification i) et le transport pneumatique ii) est inférieur à une heure.

**2.** Procédé selon la revendication 1, **caractérisé en ce que** les particules polymères absorbant l'eau sont postréticulées en surface par formation de liaisons covalentes.

**3.** Procédé selon la revendication 2, **caractérisé en ce qu'**on utilise en plus des cations métalliques polyvalents lors de la postréticulation.

**4.** Procédé selon l'une quelconque des revendications 1 à 3, **caractérisé en ce que** les particules polymères absorbant l'eau introduites dans la posthumidification i) présentent une température de 40 à 80°C.

**5.** Procédé selon l'une quelconque des revendications 1 à 4, **caractérisé en ce qu'**on utilise, pour la posthumidification i), une solution aqueuse ou une dispersion aqueuse, contenant une substance particulaire inorganique, une substance organique dissoute de manière colloïdale, un polymère organique, un polymère cationique et/ou un sel d'un cation métallique polyvalent.

**6.** Procédé selon l'une quelconque des revendications 1 à 5, **caractérisé en ce que** les particules polymères absorbant l'eau introduites dans le transport pneumatique ii) présentent une température de 40 à 80°C.

**7.** Procédé selon l'une quelconque des revendications 1 à 6, **caractérisé en ce que** la vitesse de démarrage lors du transport pneumatique ii) correspond à un nombre de Froude de 10 à 18.

**8.** Procédé selon l'une quelconque des revendications 1 à 7, **caractérisé en ce qu'**au moins 95% en poids des particules polymères absorbant l'eau présentent une grosseur de particules d'au moins 150 $\mu$m.

9. Procédé selon l'une quelconque des revendications 1 à 8, **caractérisé en ce qu'**au moins 95% en poids des particules polymères absorbant l'eau présentent une grosseur de particule d'au plus 600 μm.

10. Procédé selon l'une quelconque des revendications 1 à 9, **caractérisé en ce que** les particules polymères absorbant l'eau présentent une capacité de rétention dans une centrifugeuse d'au moins 15 g/g.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9849221 A1
- EP 0780424 A1
- WO 2007104657 A2
- WO 2007104673 A2
- WO 2007104676 A1
- US 20050013992 A
- US 20050222344 A
- EP 2135669 A1
- DE 10239074 A1
- US 6831122 B
- WO 2005080479 A1
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1

- WO 9015830 A1
- WO 2002032962 A2
- WO 2001038402 A1
- DE 3825366 A1
- US 6241928 B
- WO 2008040715 A2
- WO 2008052971 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1
- EP 0640330 A1

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 71-103

- **H. KALMAN.** *Powder Technology,* 1999, vol. 104, 214-220